# EUROPÄISCHE PATENTSCHRIFT

(11) **EP 2 298 209 B1**
(45) Veröffentlichungstag und Bekanntmachung des Hinweises auf die Patenterteilung: **06.11.2013**
(21) Anmeldenummer: 10009170.1
(22) Anmeldetag: 03.09.2010
(51) Int. Cl.: A61B 18/14, A61B 18/00

(54) **Medizinischer Resektor mit rotierbarer Hochfrequenz-Elektrode und Antriebseinheit dafür**
Medical resector with rotating high frequency electrode and drive unit for same
Dispositif de résection médical doté d'une électrode haute fréquence rotative et unité d'entraînement correspondante

(30) Priorität: 17.09.2009 DE 102009041605
(43) Veröffentlichungstag der Anmeldung: 23.03.2011
(73) Patentinhaber: Karl Storz GmbH & Co. KG, 78532 Tuttlingen (DE)
(72) Erfinder: Hamou, Jacques, Dr., 75015 Paris (FR); Simmen, Markus, 8603 Schwerzenbach (CH); Stillhard, Ottmar, 8266 Steckborn (CH)
(74) Vertreter: Fugmann, Winfried

(56) Entgegenhaltungen:
- EP-A1- 0 448 857
- WO-A1-2006/048199
- DE-A1- 3 313 325
- DE-U- 1 759 256
- FR-A1- 2 645 008
- US-B1- 6 565 561

## Beschreibung

Medizinischer Resektor mit rotierbarer Hochfrequenz-Elektrode und Antriebseinheit dafür

Die vorliegende Erfindung bezieht sich auf einen medizinischen Resektor mit einer rotierenden Hochfrequenz-Elektrode und eine Antriebseinheit für einen medizinischen Resektor mit einer rotierenden Hochfrequenz-Elektrode.

In der Hochfrequenz-Chirurgie (HF-Chirurgie) wird zum Schneiden von Gewebe eine mit hochfrequenter elektrischer Leistung versorgte Elektrode verwendet. Zum Abtragen von Gewebe wird beispielsweise eine Hochfrequenz-Elektrode aus einer Drahtschlinge verwendet. Die Schlinge wird vom Operateur durch das Gewebe geschoben oder gezogen, um Gewebeschnipsel oder Gewebespäne nahezu beliebiger Länge herauszuschneiden.

Ein von Jacques Hamou entwickelter medizinischer Resektor weist eine rotierende Drahtschlinge als Hochfrequenz-Elektrode auf. Die Drahtschlinge trägt das Gewebe in Form kleiner Späne bzw. Schnipsel ab, ähnlich beispielsweise der Funktion eines Fräskopfes zur spanabhebenden Bearbeitung von Werkstücken. Derartige medizinische Resektoren sind beispielsweise in der WO 2006/048199 A1 und der DE 10 2006 039 696 A1 beschrieben. Die Resektion bzw. die Ablation bzw. der Abtrag des Gewebes in kleinen Stücken ermöglicht eine unmittelbare Sichtkontrolle, eine vereinfachte Handhabung und einen saubereren und genaueren Abtrag von Gewebe. Außerdem können die kleinen Gewebeteile leicht entfernt werden, beispielsweise durch eine in den medizinischen Resektor integrierte Absaugung.

Angetrieben werden die rotierbaren Hochfrequenz-Elektroden herkömmlicher medizinischer Resektoren von Standardmotoren, die in der Chirurgie und anderen Bereichen der Medizin für verschiedenste Anwendungen eingesetzt werden. Diese Standardmotoren sind in der Regel mit einer INTRA-Kupplung nach ISO 3964 versehen und stellen Drehfrequenzen von mehreren zehntausend Umdrehungen pro Minute bereit. Zwischen dem Standardmotor und dem medizinischen Resektor wird deshalb ein Untersetzungs- bzw. Reduktionsgetriebe verwendet.

Ein medizinischer Resektor im Sinne der folgenden Beschreibung kann mit einem Endoskop zu einem Resektoskop kombiniert werden, wobei ein Schaft des Endoskops in einem Kanal im Schaft des medizinischen Resektors angeordnet ist.

In US 6,565,561 B1 ist ein elektrochirurgisches Instrument zur Behandlung von Gewebe in Gegenwart eines elektrisch leitfähigen Fluids beschrieben. Ein Motor erzeugt über eine Kopplungsanordnung eine Drehbewegung eines Stabs, an dessen distalem Ende eine aktive bzw. Behandlungs-Elektrode vorgesehen ist. Ein Hochfrequenzgenerator ist über ein Kabel und einen Gleitring mit dem Stab elektrisch leitfähig verbunden. Als typischer Bereich von Drehgeschwindigkeiten sind 100 Umdrehungen pro Minute bis 1000 Umdrehungen pro Minute angegeben.

In FR 2 645 008 ist eine Vorrichtung zur Resektion von weichem und hartem Gewebe, insbesondere zur Resektion der Prostata, beschrieben. Die Vorrichtung umfasst eine Einrichtung zur Geweberesektion, die einen Bogen am vorderen oder freien Ende eines Stabs bzw. rotierenden Schafts umfasst. Das hintere Ende des rotierenden Schafts ist über eine Welle mit einem Motor verbunden. Als Rotationsgeschwindigkeit sind insbesondere einige Dutzend oder einige Hundert Umdrehungen pro Minute angegeben.

Eine Aufgabe der vorliegenden Erfindung besteht darin, eine verbesserte Antriebseinrichtung für eine rotierbare Hochfrequenz-Elektrode eines medizinischen Resektors und einen verbesserten medizinischen Resektor zu schaffen.

Diese Aufgabe wird durch die Gegenstände der unabhängigen Ansprüche gelöst.

Weiterbildungen sind in den abhängigen Ansprüchen angegeben.

Verschiedene Ausfürhrungsformen der vorliegenden Erfindung beruhen auf der Idee, eine Antriebseinheit für eine rotierbare Hochfrequenz-Elektrode eines medizinischen Resektors zum Schneiden, Abtragen oder Koagulieren von menschlichem oder tierischem Gewebe zu schaffen, die eine Motoreinrichtung zum Erzeugern einer Rotationsbewegung und eine Kupplungseinrichtung zur Übertragung der von der Motoreinrichtung erzeugten Rotationsbewegung an eine mit der rotierbaren Hochfrequenz-Elektrode gekoppelte Welle, wenn die Antriebseinheit mit dem medizinischen Resektor gekoppelt ist, umfasst, wobei die Antriebseinheit ausgebildet ist, um an der Kupplungseinrichtung eine Drehfrequenz in einem Drehfrequenzbereich bereitzustellen, der im Bereich von 10 Umdrehungen pro Minute bis 200 Umdrehungen pro Minute enthalten ist.

Bei dieser Antriebseinheit sind insbesondere in einem einzigen Gehäuse alle Funktionalitäten integriert, um die Drohfrequenz bereitzustellen, mit der die rotierbare Hochfrequenz-Elektrode eines medizinischen Resektors rotieren soll. Zwar mag die Verwendung eines in nahezu jeder Klinik und nahezu jeder einschlägigen Arztpraxis vorhandenen Standardmotors attraktiv erscheinen. Tatsächlich werden dadurch die zur Verwendung eines medizinischen Resektors mit rotierbarer Hochfrequenz-Elektrode erforderlichen Investitionen verringert und im Fall eines Ausfalls des Standardmotors ein rascher und unproblematischer Ersatz durch einen anderen Standardmotor ermöglicht. Erste Erfahrungen mit der vorliegenden Erfindung zeigen jedoch, dass diese Vorteile überraschend bei weitem überwogen werden durch die wesentlich vereinfachte Handhabbarkeit einer Antriebseinheit, die ohne erst mit einem separaten Reduktionsgetriebe kombiniert werden zu müssen, unmittelbar mit einem medizinischen Resektor und dessen rotierbarer Hochfrequenz-Elektrode verbunden werden kann. Diese Verbesserung der Handhabbarkeit bezieht sich nicht nur auf die Handhabung während ihres Einsatzes, sondern vor allem auch auf die Reinigung, Wartung und Desinfektion oder Sterilisation zwischen zwei Einsätzen.

Die kompakte Antriebseinheit weist insbesondere nur eine mechanische Kupplung zur unmittelbaren Kopplung mit einer Welle einer rotierbaren Hochfrequenz-Elektrode auf, die gegen das Eindringen von Fluiden oder Fremdkörpern geschützt, gereinigt und gewartet werden muss. Weitere Kupplungen, wie sie beispielsweise zwischen einem Standardmotor und einem Reduktionsgetriebe erforderlich sind, entfallen. Auch das Erfordernis einer lösbaren aber ausreichend robusten mechanischen Verbindung zwischen Standardmotor und Reduktionsgetriebe entfällt. Ferner kann die Antriebseinheit aufgrund des Wegfalls von Kupplungen und anderer mechanischen Schnittstellen je nach vorgesehener Anwendung viel kompakter, leichter und kostengünstiger herstellbar sein als eine herkömmliche Kombination aus Standardmotor und externem Reduktionsgetriebe.

Zur Ermittlung geeigneter Drehfrequenzen bzw. Drehzahlen bzw. Rotationsfrequenzen wurden zunächst theoretische Überlegungen und Berechnungen angestellt, die von Erfahrungswerten mit herkömmlichen, nicht rotierenden schlingenförmigen Hochfrequenz-Elektroden ausgehen. Für derartige herkömmliche Hochfrequenz-Elektroden kann abhängig vom Gewebe und anderen Umständen des Einzelfalls eine Schnittgeschwindigkeit von ca. 10 Millimeter pro Sekunde als bewährt angesehen werden. Es wird davon ausgegangen, dass die Schnittgeschwindigkeit an keiner Stelle an der rotierenden Hochfrequenz-Elektrode über der bewährten Schnittgeschwindigkeit von 10 Millimeter pro Sekunde liegen soll.

Die Schnittgeschwindigkeit ist außer von der Drehfrequenz auch vom Radius linear abhängig. Berechnungen ergeben bei einer maximalen Schnittgeschwindigkeit von 10 Millimeter pro Sekunde und bei einem Durchmesser der Drahtschlinge bzw. der Hochfrequenz-Elektrode von 4 Millimetern eine Drehfrequenz von 48 Umdrehungen pro Minute, bei 6 Millimetern eine Drehfrequenz von 32 Umdrehungen pro Minute und bei 8 Millimetern eine Drehfrequenz von 24 Umdrehungen pro Minute. Umfangreiche, detaillierte und deshalb auch langwierige experimentelle Untersuchen durch Jacques Hamou bestätigen, dass bei den üblichen Durchmessern der Hochfrequenz-Elektrode bzw. der Drahtschlinge bei Drehfrequenzen zwischen 30 Umdrehungen pro Minute und 60 Umdrehungen pro Minute überraschend nicht nur hinsichtlich der Schnittleistung und der Schnittqualität erhebliche Vorteile erzielt werden. Abhängig von der Größe und Form der Hochfrequenz-Elektrode sowie vom zu schneidenden Gewebe sind auch niedrigere Drehfrequenzen ab etwa 10 Umdrehungen pro Minute oder 20 Umdrehungen pro Minute und höhere Drehfrequenzen bis ca. 100 Umdrehungen pro Minute, im Einzelfall bis 150 Umdrehungen pro Minute oder 200 Umdrehungen pro Minute, vorteilhaft.

Überraschend liegt bei den genannten Drehfrequenzen, insbesondere bei Drehfrequenzen zwischen 30 Umdrehungen pro Minute und 60 Umdrehungen pro Minute, ein Optimum vor hinsichtlich der Abtragsleistung einerseits sowie der Beobachtbarkeit und Kontrollierbarkeit des Abtrags andererseits. Trotz einer guten Abtragleistung sind die abgetragenen Gewebestücke so klein, dass sie die Sicht durch ein beispielsweise im Resektor angeordnetes Endoskop auf den Arbeitsbereich nur unwesentlich beeinträchtigen. Die geringe Beeinträchtigung der Sicht durch das Endoskop rührt auch daher, dass die Gewebestücke schnell und ohne das Risiko einer Verstopfung des medizinischen Resektors abgesaugt und damit aus dem Blickfeld entfernt werden können.

Überraschend spielt die Drehfrequenz der rotierbaren Elektrode auch für die Belastung des medizinischen Personals und damit wiederum indirekt für die Qualität des erzielten Ergebnisses eine wichtige Rolle. Bei den genannten Drehfrequenzen, besonders bei Drehfrequenzen bis 60 Umdrehungen pro Minute, kann die Bewegung der Hochfrequenz-Elektrode vom Auge des medizinischen Personals noch ohne Weiteres wahrgenommen und aufgelöst werden. Die Beobachtung der Hochfrequenz-Elektrode wird deshalb noch nicht als anstrengend wahrgenommen. Jedes einzelne abgetragene Gewebestück und die dadurch freigelegten tieferen Gewebeschichten und deren Oberflächen können verfolgt bzw. beobachtet werden. Bei deutlich höheren Drehfrequenzen über 100 Umdrehungen pro Minute und noch mehr bei über 200 Umdrehungen pro Minute wirkt die Bewegung der Hochfrequenz-Elektrode und der Gewebestücke und ihre Beobachtung zunehmend ermüdend. Dies ist bei medizinischen Anwendungen nicht nur in Rücksicht auf das medizinische Personal sondern vor allem auch auf die Patienten gefährlich.

Ferner ist bei den genannten Drehfrequenzen, insbesondere im Bereich von 30 Umdrehungen pro Minute bis 60 Umdrehungen pro Minute, der Abtrag besonders gut kontrollierbar. Die beschriebene gute Beobachtbarkeit einerseits und der ausreichend schnelle aber nicht zu schnelle Abtrag sind offenbar besonders geeignet für eine Steuerung und Regelung des Abtrags durch medizinisches Personal.

Die genannten Vorteile werden so und vor allem auch in dieser Kombination bei keiner anderen Drehfrequenz erzielt. Niedrigere Drehfrequenzen haben eine unzureichende Abtragsleistung zur Folge. Außerdem werden die einzelnen abgetragenen Gewebestücke zu groß, um einwandfrei abgesaugt zu werden. Ferner verdecken die Gewebestücke die freigelegten Gewebeschichten zu lange bevor sie ganz abgelöst und beispielsweise durch Absaugen aus dem Blickfeld entfernt sind.

Bei höheren Drehfrequenzen über 200 Umdrehungen pro Minute, je nach Gewebe oft schon bei Drehfrequenzen über 100 Umdrehungen pro Minute oder über 60 Umdrehungen pro Minute, ist die Qualität der Schnitte, insbesondere die Koagulation, oft unzureichend. Hinzu kommt, dass die Beobachtung der schnellen Bewegungen für das Auge des medizinischen Personals ermüdend ist. Je nach Gewebe können ferner die abgetragenen Gewebestücke zu klein sein, wodurch sie insbesondere an Oberflächen haften und nur noch schwer abzusaugen sind.

Auch für die Vorschubgeschwindigkeit wurden entsprechende Berechnungen angestellt. Bei einer Drehfrequenz von 50 Umdrehungen pro Minute und einem Vorschub von 5 Millimetern pro Sekunde resultiert eine Schnittiefe bzw. eine Spandicke von 3 Millimetern. Überlegungen und Untersuchungen zur Form einer schlingenförmigen Hochfrequenz-Elektrode führten zu einer kreisförmigen oder elliptischen oder ovalen Form, wobei die Schlinge in einer Ebene liegt. Bei einer elliptischen oder ovalen Form ist die kleine Hauptachse parallel zur Rotationsachse angeordnet, insbesondere fällt die kleine Hauptachse mit der Rotationsachse zusammen.

Um an der Kupplungseinrichtung eine Drehfrequenz in einem Drehfrequenzbereich bereitzustellen, der im Bereich von 10 Umdrehungen pro Minute bis 200 Umdrehungen pro Minute enthalten ist, weist die oben beschriebene Antriebseinheit insbesondere ein integriertes Reduktionsgetriebe bzw. Untersetzungsgetriebe auf. Das Reduktionsgetriebe und der Motor sind insbesondere fest an einander geflanscht oder weisen ein gemeinsames Gehäuse auf. Die Integration eines Reduktionsgetriebes in die Antriebseinheit ermöglicht eine optimale Anpassung desselben an den Motor. Bei Verwendung eines Motors, der die erforderliche Leistung bei einer Drehfrequenz von wenigen tausend Umdrehungen pro Minute abgibt, ist eine deutlich geringere Untersetzung erforderlich als bei den herkömmlich verwendeten Standardmotoren mit etwa zehnfach höherer Drehfrequenz.

Alternativ weist die Antriebseinheit einen Motor auf, der die erforderliche Leistung bei den genannten Drehfrequenzen bereitstellt, so dass kein Untersetzungsgetriebe erforderlich ist. Dazu ist neben einem langsam laufenden elektrischen, hydraulischen oder pneumatischen Motor insbesondere ein Ultraschallmotor verwendbar. Der gänzliche Verzicht auf ein Reduktionsgetriebe ist hinsichtlich der Größe und der Masse der Antriebseinheit sowie hinsichtlich des im Betrieb erzeugten Geräusches optimal und reduziert das Spiel auf ein Minimum.

Die Kupplungseinrichtung kann einen Hohlraum mit einem zylindrischen (aber in der Regel nicht kreiszylindrischen) Abschnitt, der nicht zur Rotationsachse der Kupplungseinrichtung symmetrisch ist, umfassen. Ein zylindrischer Abschnitt ist ein Abschnitt, der zylindrisch oder im Wesentlichen zylindrisch ist. Der zylindrische Abschnitt ist dann nicht zur Rotationsachse der Kupplungseinrichtung symmetrisch, wenn Abweichungen von der Symmetrie nicht nur durch Nocken oder dergleichen begründet werden, wie sie teilweise bei herkömmlichen Kupplungseinrichtungen Verwendung als Mitnehmer oder zur Verrastung finden.

In diesen zylindrischen Hohlraum kann ein entsprechend gestalteter Abschnitt an einer korrespondierenden Kupplungseinrichtung des medizinischen Resektors eingreifen. Der zylindrische Hohlraum weist beispielsweise einen Querschnitt auf, dessen Rand einen geraden Abschnitt umfasst.

Beispielsweise weist der Rand eines halbkreisförmigen Querschnitts einen geraden Abschnitt auf. Eine Kupplungseinrichtung mit einem derartigen Querschnitt besteht beispielsweise aus einem Rohr mit einem Lumen mit kreisförmigem Querschnitt, wobei in das Lumen des Rohrs ein halbkreiszylindrischer Körper eingesetzt ist. Der halbkreiszylindrische Körper weist einen halbkreisförmigen Querschnitt mit einem kreisbogenförmigen Randabschnitt und einem geraden Randabschnitt auf. Der kreisbogenförmige Abschnitt des Rands des Querschnitts dieses Körpers hat den gleichen Radius wie der Querschnitt des Lumens des Rohrs und liegt am Rand des Lumens an. Dann ist der Querschnitt des im Lumen des Rohrs verbleibenden Hohlraums ebenso halbkreisförmig wie der Querschnitt des in das Lumen eingesetzten Körpers, aber gegenüber diesem um 180° versetzt. Wenn die Symmetrieachse des Lumens des Rohrs gleichzeitig die Rotationsachse der Kupplungseinrichtung ist, liegt die Mitte des geraden Abschnitts des Rands des Querschnitts des Hohlraums auf der Rotationsachse der Kupplungseinrichtung.

Etwas verallgemeinert weist der Rand des Querschnitts des zylindrischen Hohlraums einen Abschnitt auf, der zum Schnittpunkt der Rotationsachse mit dem Rand des Querschnitts punktsymmetrisch ist. Beispielsweise weist der zylindrische Hohlraum einen halbelliptischen, rechteckigen oder dreieckigen Querschnitt auf, wobei der punktsymmetrische Abschnitt auch in diesen Fällen jeweils gerade ist. Der punksymmetrische Abschnitt des Rands des Querschnitts kann aber auch beispielsweise S-förmig sein.

Ferner kann der Querschnitt des zylindrischen Hohlraums eine beliebige Form aufweisen. Zur formschlüssigen Übertragung eines Drehmoments ist der Querschnitt nicht-kreisförmig. Eine Übertragung eines Drehmoments ist auch bei einem kreisförmigen Querschnitt möglich, wenn der Mittelpunkt des kreisförmigen Querschnitts nicht auf der Rotationsachse der Kupplungseinrichtung liegt.

Die beschriebenen Ausführungsformen der Kupplungseinrichtung weisen abhängig von dem Material der Kupplungseinrichtung, ihrem Herstellungsverfahren, dem zu übertragenden Drehmoment und anderen Parametern eine Reihe von Vorteilen auf. Insbesondere können sie in vielen Fällen besonders einfach und damit kostengünstig hergestellt werden. Die genannte Punktsymmetrie und insbesondere der halbkreisförmige Querschnitt schaffen auf besonders einfache Weise eine kostengünstig herstellbare Kupplungseinrichtung mit geringem Spiel.

Die Antriebseinheit kann ausgebildet sein, um mit dem medizinischen Resektor - insbesondere mit einem Schaft oder einer Abflusskammer des medizinischen Resektors - so verbunden zu werden, dass eine Rotationsachse der Kupplungseinrichtung zu einer Längsachse des medizinischen Resektors, insbesondere des Schafts des medizinischen Resektors - nicht parallel ist. Diese Nicht-Parallelität umfasst insbesondere eine Abweichung von der Parallelität, die über die in der Medizintechnik übliche Präzision bzw. über die in der Medizintechnik üblichen Toleranzen hinausgeht. Insbesondere liegt zwischen der Längsachse des medizinischen Resektors bzw. seines Schafts einerseits und der Rotationsachse der Kupplungseinrichtung der Antriebseinheit andererseits ein Winkel von mindestens 0,4°, insbesondere ein Winkel zwischen 0,6° und 1,0° vor.

Wie bereits erwähnt ist die Kupplungseinrichtung an der Antriebseinheit zur Übertragung der von der Motoreinrichtung erzeugten Rotationsbewegung an eine mit der rotierbaren Hochfrequenz-Elektrode gekoppelte Welle an einem Resektor ausgebildet. Dazu ist am proximalen Ende der Welle eine zu der Kupplungseinrichtung der Antriebseinheit korrespondierende Kupplungseinrichtung vorgesehen. Letztere wird nachfolgend als resektorseitige Kupplungseinrichtung bezeichnet.

Der genannte Winkel zwischen der Rotationsachse der Kupplungseinrichtung der Antriebseinheit einerseits und der Längsachse eines mit der Antriebseinheit zu koppelnden Resektors andererseits ist insbesondere angepasst an einen Winkel zwischen der Rotationsachse der rotierbaren Hochfrequenz-Elektrode bzw. der Welle zwischen Hochfrequenz-Elektrode und der resektorseitigen Kupplungseinrichtung einerseits und der Längsachse des medizinischen Resektors bzw. seines Schafts andererseits. Die Winkel sind insbesondere so angepasst dass die Rotationsachse der Kupplungseinrichtung der Antriebseinheit und die Rotationsachse der resektorseitigen Kupplungseinrichtung zusammenfallen.

Ein solcher Winkel bzw. eine solche Nicht-Parallelität an einem medizinischen Resektor ermöglicht eine zentrale Anordnung der rotierbaren Hochfrequenz-Elektrode am distalen Ende einerseits und gleichzeitig eine exzentrische Anordnung des Endes der Welle und der daran angeordneten resektorseitigen Kupplungseinrichtung am proximalen Ende des medizinischen Resektors andererseits. Die zentrale Anordnung der rotierbaren Hochfrequenz-Elektrode ist unter anderem deshalb vorteilhaft, weil die Hochfrequenz-Elektrode damit einen maximalen Durchmesser haben kann, ohne über die Kontur des Schafts des medizinischen Resektors überzustehen. Die exzentrische Anordnung der Welle und der resektorseitigen Kupplungseinrichtung am proximalen Ende schafft einen maximalen Abstand beispielsweise von einem in den Schaft des medizinischen Resektors einführbaren Schaft eines Endoskops und damit maximalen Bauraum für die Antriebseinheit.

Eine Koaxialität zwischen der Rotationsachse der rotierbaren Hochfrequenz-Elektrode, der Welle und der resektorseitigen Kupplungseinrichtung einerseits und der Rotationsachse der Kupplungseinrichtung der Antriebseinheit andererseits ermöglicht eine besonders reibungsarme und damit verlust- und verschleißarme Auslegung. Bei entsprechender Ausgestaltung der Kupplungseinrichtungen von Antriebseinheit und medizinischem Resektor kann ein kleine Winkeldifferenz bis zu wenigen Grad durch die Kupplungseinrichtungen ausgeglichen werden. In diesem Fall kann trotz einer nicht-parallelen Anordnung der Rotationsachse der Welle des medizinischen Resektors zur Längsachse des medizinischen Resektors die Antriebseinheit für eine Anordnung ausgebildet sein, bei der die Rotationsachse der Kupplungseinrichtung der Antriebseinheit parallel zur Längsachse des medizinischen Resektors ist.

Eine Antreibseinrichtung, wie sie oben beschrieben wurde, kann ferner eine Verriegelungseinrichtung zum Verriegeln der Kupplungseinrichtung des medizinischen Resektors in der Antriebseinheit aufweisen. Die Verriegelungseinrichtung ist insbesondere ausgebildet, um nicht mit der Kupplungseinrichtung zu rotieren. Die Verriegelungseinrichtung umfasst insbesondere einen federbelasteten Riegel, der ausgebildet ist, um in eine Nut an der Welle einzugreifen. Diese Verriegelungseinrichtung kann unmittelbar distal angrenzend an die Kupplungseinrichtung angeordnet sein. Dies ermöglicht nicht nur eine besonders kompakte Bauweise, sondern reduziert auch das Spiel und den Einfluss von Elastizitäten auf das mechanische Zusammenspiel der Kupplungseinrichtung der Antriebseinheit einerseits und einer resektorseitigen Kupplungseinrichtung andererseits. Sowohl die Verriegelungseinrichtung als auch die Kupplungseinrichtung können dadurch besonders präzise und spielfrei wirken.

Der federbelastete Riegel ist insbesondere in Form eines Schiebers ausgebildet. Dieser Schieber kann an beiden Enden einen kreisförmigen Querschnitt aufweisen und so in einer entsprechenden Bohrung präzise geführt sein. In einem mittleren Bereich ist er beispielsweise quaderförmig mit einem Loch, einer Öffnung oder einer Ausnehmung. Das Loch, die Öffnung oder die Ausnehmung sind so gestaltet, dass zumindest in einer Position des Riegels eine Kupplungseinrichtung eines medizinischen Resektors hindurchgeführt werden kann. Wenn die resektorseitige Kupplungseinrichtung in der vorgesehenen Weise in die Kupplungseinrichtung der Antriebseinheit eingreift, kann ein Rand des Lochs bzw. der Öffnung bzw. der Ausnehmung des Riegels in eine umlaufende Nut an der resektorseitigen Kupplungseinrichtung eingreifen. Dadurch verriegelt der Riegel die resektorseitige Kupplungseinrichtung in der Kupplungseinrichtung der Antriebseinheit. Durch die Federbelastung wird der Riegel in dieser Verriegelungsposition gehalten solange er nicht manuell gegen die Federbelastung in eine Position geschoben wird, in der die resektorseitige Kupplungseinrichtung wieder herausgezogen werden kann.

Die beschriebene Verriegelungseinrichtung mit einem federbelasteten Riegel kann abhängig von den Anforderungen, die zu erfüllen sind, besonders einfach und kostengünstig herstellbar und gleichzeitig robust und präzise ausgeführt werden.

Eine Kupplungseinrichtung einer Antriebseinheit, wie sie oben beschrieben wurde, kann ferner ausgebildet sein, um ein elektrisches Signal, insbesondere ein elektrisches Hochfrequenzsignal und damit elektrische Hochfrequenzleistung, von der Antriebseinheit zu der Welle eines mit der Antriebseinheit gekoppelten medizinischen Resektors zu übertragen. Die beschriebene Antriebseinheit kann damit mehrere Funktionen gleichzeitig erfüllen, insbesondere gleichzeitig eine Rotationsbewegung und ein elektrisches Signal auf die Welle des medizinischen Resektors und über die Welle zur rotierbaren Hochfrequenz-Elektrode übertragen. Dies setzt eine entsprechend ausgebildete resektorseitige Kupplungseinrichtung voraus. Insbesondere weisen beide Kupplungseinrichtungen ein Metall oder ein anderes elektrisch leitfähiges Material auf. Die Notwendigkeit, das elektrische Signal am medizinischen Resektor einzuspeisen und am medizinischen Resektor eine Einrichtung zum Übertragen eines elektrischen Signals an die Welle vorzusehen, entfällt damit.

Zur Übertragung des elektrischen Signals zu der Kupplungseinrichtung ist insbesondere eine Kontaktierungseinrichtung an oder in der Antriebseinheit vorgesehen. Die Kontaktierungseinrichtung umfasst einen oder mehrere Schleifkontakte, die an einer Mantelfläche der Kupplungseinrichtung anliegen. Die Anordnung der Schleifkontakte an der Mantelfläche der Kupplungseinrichtung kann eine besonders kompakte Bauform der gesamten Antriebseinheit fördern. In kleinstem Bauraum sind damit eine Kupplungseinrichtung, eine Verriegelungseinrichtung und eine Kontaktierungseinrichtung realisierbar. Anstelle einer Kontaktierungseinrichtung mit Schleifkontakten ist eine Übertragung des elektrischen Signals über die oben beschriebene Verriegelungseinrichtung direkt an die resektorseitige Kupplungseinrichtung möglich.

Die Antriebseinheit kann ausgebildet sein, um mit einer Abflusskammer am proximalen Ende des Schafts eines medizinischen Resektors unmittelbar, fluiddicht und lösbar verbunden zu werden. Dazu weist die Antriebseinheit insbesondere eine zur Geometrie der Abflusskammer korrespondierende Geometrie und eine Dichteinrichtung auf. Die unmittelbare, fluiddichte und lösbare Verbindung der Antriebseinheit mit der Abflusskammer ermöglicht einen besonders einfachen und kostengünstig herstellbaren Aufbau des medizinischen Resektors und eine besonders geringe Baulänge.

Ein medizinischer Resektor zum Schneiden, Abtragen oder Koagulieren von menschlichem oder tierischem Gewebe umfasst eine rotierbare Hochfrequenz-Elektrode am distalen Ende eines Schafts des medizinischen Resektors, eine Kupplungseinrichtung am proximalen Ende des Schafts und eine Welle, welche die Kupplungseinrichtung mit der rotierbaren Hochfrequenz-Elektrode verbindet. Die Kupplungseinrichtung ist zur lösbaren mechanischen Kopplung mit einer Antriebseinheit, insbesondere einer Antriebseinheit, ausgebildet, die eine Drehfrequenz in einem Drehfrequenzbereich bereitstellt, der im Bereich von 10 Umdrehungen pro Minute bis 200 Umdrehungen pro Minute enthalten ist. Die Kupplungseinrichtung ist insbesondere zur lösbaren mechanischen Kopplung mit einer der oben beschriebenen Antriebseinheit ausgebildet.

Der beschriebene medizinische Resektor ermöglicht, insbesondere zusammen mit der oben beschriebenen Antriebseinheit, einen besonders einfachen und kostengünstig herstellbaren Aufbau und eine besonders geringe Baulänge. Vorteile der oben beschriebenen Antriebseinheit gelten auch für eine Kombination aus der Antriebseinheit und dem medizinischen Resektor, teilweise auch für den medizinischen Resektor alleine.

Die Kupplungseinrichtung des medizinischen Resektors kann einen zylindrischen Abschnitt umfassen, der nicht zur Rotationsachse der Kupplungseinrichtung symmetrisch ist. Ein zylindrischer Abschnitt ist ein Abschnitt, der zylindrisch oder im Wesentlichen zylindrisch ist. Der zylindrische Abschnitt ist dann nicht zur Rotationsachse der Kupplungseinrichtung symmetrisch, wenn Abweichungen von der Symmetrie nicht nur durch Nocken oder dergleichen begründet werden, wie sie teilweise bei herkömmlichen Kupplungseinrichtungen Verwendung als Mitnehmer oder zur Verrastung finden.

Der zylindrische Abschnitt kann in einen entsprechend gestalteten Abschnitt eines Hohlraums an einer korrespondierenden Kupplungseinrichtung des medizinischen Resektors eingreifen. Der zylindrische Abschnitt der Kupplungseinrichtung des medizinischen Resektors weist beispielsweise einen Querschnitt auf, dessen Rand einen Abschnitt umfasst, der gerade oder zum Schnittpunkt der Rotationsachse mit dem Rand des Querschnitts punktsymmetrisch ist. Beispielsweise weist der zylindrische Abschnitt einen halbkreisförmigen, halbelliptischen, rechteckigen oder dreieckigen Querschnitt auf.

Die Rotationsachse der Kupplungseinrichtung des medizinischen Resektors kann auf einem Abschnitt des Rands des Querschnitts des Hohlraums der Kupplungseinrichtung liegen, der gerade oder zum Schnittpunkt der Rotationsachse mit dem Rand des Querschnitts punktsymmetrisch ist.

Die Welle des medizinischen Resektors kann eine Rotationsachse aufweisen, die zu einer Längsachse des medizinischen Resektors bzw. eines Schafts des medizinischen Resektors nicht parallel ist.

Ein medizinischer Resektor, wie er oben beschrieben ist, kann eine der oben beschriebenen Antriebseinheiten umfassen. Ferner kann der medizinische Resektor mit einem Endoskop zu einem Resektoskop kombiniert werden, wobei ein Schaft des Endoskops in einem Kanal im Schaft des medizinischen Resektors angeordnet ist.

### Kurzbeschreibung der Figuren

Nachfolgend werden Ausführungsformen anhand der beigefügten Figuren näher erläutert. Es zeigen:
- Figur 1: eine schematische Darstellung eines medizinischen Resektors mit einem Endoskop;
- Figur 2: eine schematische Darstellung eines Teils einer Antriebseinheit;
- Figur 3: eine schematische Darstellung eines Teils der Antriebseinheit aus Figur 2;
- Figur 4: eine schematische Darstellung eines Teils der Antriebseinheit aus Figur 2;
- Figur 5: eine schematische Darstellung der Antriebseinheit aus Figur 2;
- Figur 6: eine schematische Darstellung von Querschnitten von Kupplungseinrichtun- gen.

### Beschreibung der Ausführungsformen

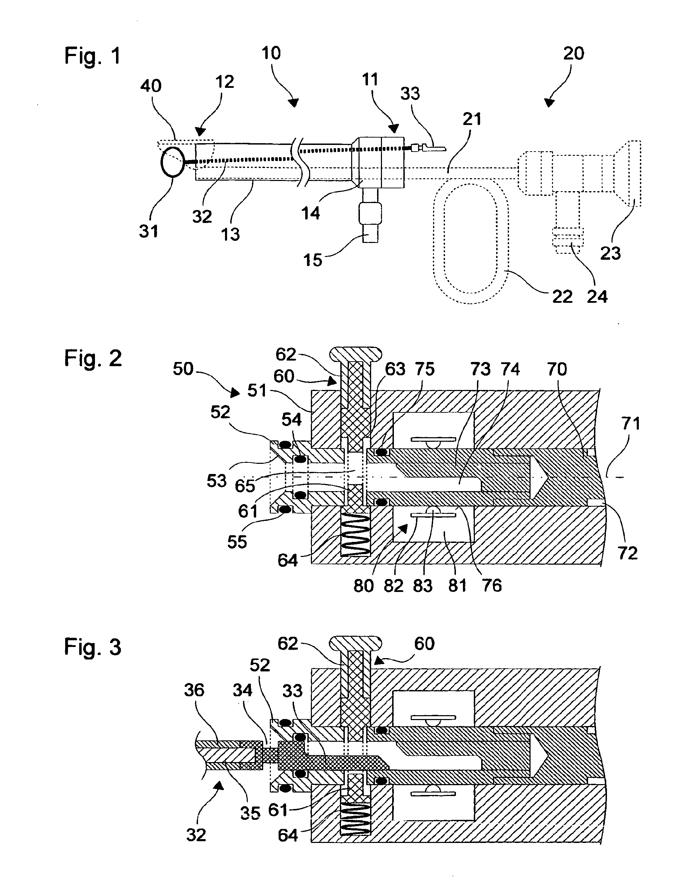

Figur 1 zeigt eine schematische Darstellung eines medizinischen Resektors 10 zum Schneiden, Abtragen oder Koagulieren von menschlichem oder tierischem Gewebe mit einem proximalen Ende 11, einem distalen Ende 12 und einem Schaft 13, der sich vom proximalen Ende 11 zum distalen Ende 12 erstreckt. Der Schaft 13 ist zylindrisch mit einem kreisförmigen, elliptischen, ovalen oder beliebigen anderen Querschnitt. Die Längsachse des Schafts 13 ist parallel zur Zeichenebene der Figur 1.

Am proximalen Ende 11 des medizinischen Resektors 10 weist dieser eine Abflusskammer 14 mit einem Anschluss 15 für eine in Figur 1 nicht dargestellte Saugeinrichtung auf. In die Abflusskammer 14 und den Schaft 13 kann ein Endoskop 20 bzw. dessen Schaft 21 eingeführt werden. Dazu kann der medizinische Resektor 10 einen entsprechenden Kanal mit Öffnungen an beiden Enden aufweisen, der sich durch die Abflusskammer 14 und den Schaft 13 vom proximalen Ende 11 bis zum distalen Ende 12 des medizinischen Resektors erstreckt.

Der medizinische Resektor 10 ist auch ohne das Endoskop 20 verwendbar. Das Endoskop 20 ist deshalb in unterbrochenen Linien dargestellt. Der medizinische Resektor 10 und das Endoskop 20 zusammen bilden jedoch eine häufig eingesetzte Kombination, die auch als Resektoskop bezeichnet wird. Durch das Endoskop kann die abzutragende oder zu koagulierende Oberfläche vor dem Einsatz und auch währen des Einsatzes des medizinischen Resektors 10 betrachtet und die Wirkung des Resektors 10 unmittelbar beobachtet werden.

Das Endoskop 20 umfasst den bereits erwähnten Schaft 21, einen die Handhabbarkeit des Endoskops 20 verbessernden Griff 22 oder eine andere Handhabe, ein Okular 23 und eine Kupplung 24 zur Verbindung des Endoskops 20 mit einer Lichtquelle, die in Figur 1 nicht dargestellt ist. Alternativ ist der Griff 22 oder eine andere Handhabe abweichend von der Darstellung in Figur 1 an einem Schaft angeordnet, in den der Schaft 21 des Endoskops eingeführt und dort insbesondere befestigt oder verriegelt werden kann. Mit dem Griff 22 oder einer anderen Handhabe am Endoskop 20 oder alternativ an einem Schaft, in dem der Schaft 21 des Endoskops 20 geführt und gehalten ist, kann das Endoskop 20 unabhängig von dem Resektor 10 geführt, beispielsweise im Resektor 10 gedreht werden.

Der medizinische Resektor 10 umfasst ferner eine rotierbare Hochfrequenz-Elektrode 31 an seinem distalen Ende 12. Die rotierbare Hochfrequenz-Elektrode 31 ist an einer Welle 32 befestigt oder mit dieser einstückig ausgeführt. Die Welle 32 erstreckt sich im Inneren des Schafts 13 des medizinischen Resektors 10 von der rotierbaren Hochfrequenz-Elektrode 31 am distalen Ende 12 bis zu einer Kupplung 33 am proximalen Ende 11. Die Kupplung 33 ist proximal der Abflusskammer 14 angeordnet. Die Welle 32 ist ausgebildet, um eine Rotation der Kupplung 33 zur Hochfrequenz-Elektrode 31 zu übertragen. Zur rotierbaren Lagerung der Welle 32 sind ein oder mehrere Lager und/oder ein Führungsrohr, das sich vom proximalen Ende 11 bis zum distalen Ende 12 des medizinischen Resektors 10 erstreckt, vorgesehen. Lager und Führungsrohr sind in Figur 1 nicht dargestellt.

Die Welle 32 und damit auch die Rotationsachse der rotierbaren Hochfrequenz-Elektrode 31, der Welle 32 und der Kupplungseinrichtung 33 sind nicht parallel zur Längsachse des Schafts 13. Die Rotationsachse und die Längsachse bilden einen Winkel von mindestens 0,4°, insbesondere einen Winkel zwischen 0,6° und 1,0° oder einen Winkel von im Wesentlichen 0,8°. Dieser Winkel ermöglicht einerseits eine zentrale Anordnung der Hochfrequenz-Elektrode 31 am distalen Ende 12 und andererseits einen maximalen Abstand zwischen der Kupplungseinrichtung 33 am proximalen Ende 11 und dem Schaft 21 des Endoskops 20.

Am distalen Ende 12 ist eine Krageneinrichtung 40 aus Kunststoff, Keramik oder einem anderen elektrisch isolierenden Material angeordnet. Die optionale Krageneinrichtung 40 schützt die Hochfrequenz-Elektrode 31 vor einer Beschädigung und den menschlichen oder tierischen Körper vor einer Verletzung durch die Hochfrequenz-Elektrode 31, insbesondere beim Einführen des medizinischen Resektors 10. Ferner kann die Krageneinrichtung 40 eine am distalen Ende 12 des medizinischen Resektors 10 austretende Flüssigkeit leiten. Zusätzlich oder alternativ kann die Krageneinrichtung 40 eine am distalen Ende 12 des medizinischen Resektors 10 erzeugte Absaugwirkung auf den Raumbereich um die Hochfrequenz-Elektrode 31 kanalisieren oder konzentrieren. In beiden Fällen verbessert die Krageneinrichtung 40 den Blick durch das Endoskop 20 auf eine mittels der Hochfrequenz-Elektrode 31 bearbeitete Oberfläche.

Bei der medizinischen Anwendung des Resektoskops aus medizinischem Resektor 10 und in diesen eingesetztem Endoskop 20 haben sich Drehfrequenzen in dem eingangs beschriebenen Bereich zwischen 30 Umdrehungen pro Minute und 60 Umdrehungen pro Minute besonders bewährt. Je nach Anwendung, Durchmesser und Form der rotierbaren Elektrode auch Drehfrequenzen ab 20 Umdrehungen pro Minute, teilweise sogar ab 10 Umdrehungen pro Minute und bis zu 100 Umdrehungen pro Minute oder 150 Umdrehungen pro Minute, teilweise sogar bis zu 200 Umdrehungen pro Minute.

Für die genannten Drehfrequenzen wurden aufgrund der oben dargestellten Überlegungen und Berechnungen gute Ergebnisse hinsichtlich der Schnittleistung und Schnittqualität erwartet. Diese und weitere, teilweise überraschende Vorteile konnten bei umfangreichen, intensiven und deshalb auch sehr langwierigen Experimenten bestätigt werden. Insbesondere liegt bei den genannten Drehfrequenzen ein Optimum vor hinsichtlich der Abtragsleistung einerseits sowie der Beobachtbarkeit und Kontrollierbarkeit des Abtrags andererseits. Die abgetragenen Gewebestücke sind so klein, dass sie die Sicht durch das Endoskop 20 auf den Arbeitsbereich nur unwesentlich beeinträchtigen, auch deshalb, weil sie schnell und ohne das Risiko einer Verstopfung des medizinischen Resektors 10 abgesaugt und damit aus dem Blickfeld entfernt werden können.

Ferner kann die Bewegung der Hochfrequenz-Elektrode vom Auge des medizinischen Personals noch ohne Weiteres wahrgenommen und aufgelöst werden. Die Beobachtung einer Hochfrequenz-Elektrode, die mit einer Drehfrequenz im genannten Bereich, insbesondere zwischen 30 Umdrehungen pro Minute und 60 Umdrehungen pro Minute, rotiert, wird deshalb noch nicht als anstrengend wahrgenommen. Jedes einzelne abgetragene Gewebestück und die dadurch freigelegten tieferen Gewebeschichten und deren Oberflächen können verfolgt bzw. beobachtet werden. Bei deutlich höheren Drehfrequenzen über 100 Umdrehungen pro Minute und noch mehr bei über 200 Umdrehungen pro Minute wirkt die Bewegung der Hochfrequenz-Elektrode und der Gewebestücke und ihre Beobachtung rasch ermüdend. Dies ist bei medizinischen Anwendungen aber nicht nur in Rücksicht auf das medizinische Personal sondern vor allem auch auf die Patienten gefährlich.

Mit dem oben anhand der Figur 1 dargestellten medizinischen Resektor 10 ist eine Antriebseinheit für die rotierbare Hochfrequenz-Elektrode 31 koppelbar, die nachfolgend mit Bezug auf die Figuren 2 bis 6 näher beschrieben wird. Jede der Figuren 2 bis 5 zeigt eine schematische Darstellung eines Schnitts durch die Antriebseinheit 50. Die in den Figuren 2 bis 5 dargestellte Schnittebene liegt senkrecht zur Zeichenebene der Figur 1. Dabei ist jeweils links das distale Ende bzw. die distale Seite der Antriebseinheit 50 dargestellt und rechts das proximale Ende bzw. die proximale Seite. Zur Verbesserung der Übersichtlichkeit der Darstellung sind nicht in jeder der Figuren 2 bis 5 alle Bezugszeichen gezeigt.

Figur 2 zeigt eine schematische Darstellung eines Schnitts eines Teils der Antriebseinheit 50. Die Antriebseinheit 50 umfasst einen Gehäusekörper 51, der beispielsweise aus Kunststoff oder einem anderen elektrisch isolierenden Material hergestellt ist. Der Gehäusekörper 51 weist mehrere Bohrungen und Hohlräume auf, von denen ein Teil in den Figuren 2 bis 5 sichtbar ist und nachfolgend beschrieben wird.

Insbesondere weist der Gehäusekörper 51 eine im Wesentlichen kreiszylindrische Längsbohrung auf, die in der Darstellung in Figur 2 von links nach rechts, also von distal nach proximal, verläuft. In das distale Ende der Längsbohrung ist eine Konuseinrichtung 52 eingesetzt. Die Konuseinrichtung 52 weist eine Konusfläche 53, eine in Figur 2 erkennbare Durchgangsbohrung und in umlaufende Nuten innen bzw. außen eingesetzte O-Ringe oder andere Dichteinrichtungen 54, 55 auf.

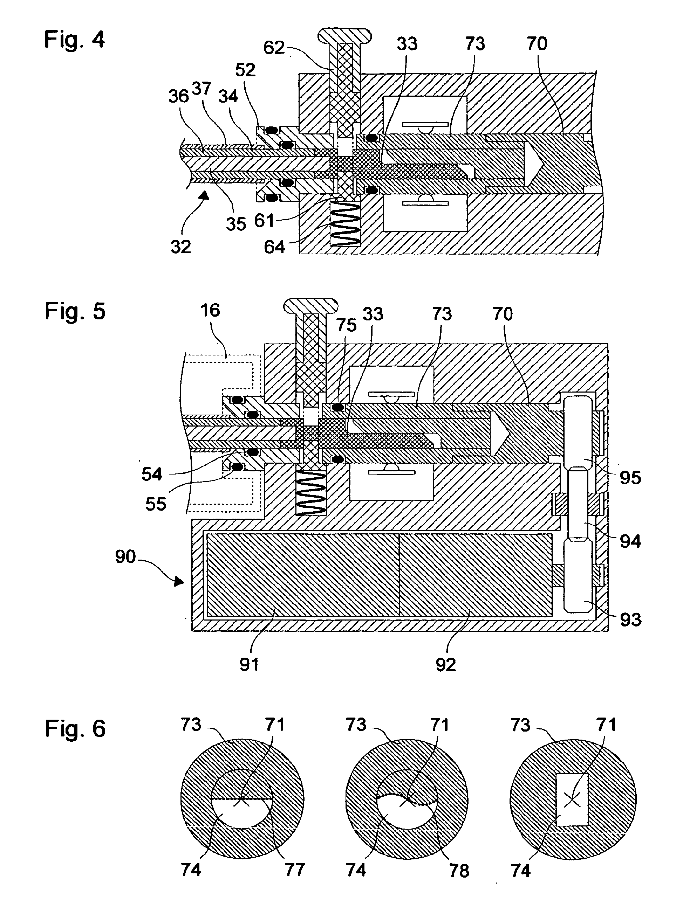

Der Gehäusekörper 51 weist ferner eine zur Längsbohrung senkrechte Querbohrung auf, die bei der Darstellung in Figur 2 von oben nach unten verläuft. In dieser zweiten Bohrung ist eine Verriegelungseinrichtung 60 angeordnet, deren Funktion weiter unten mit Bezug auf die Figuren 3 und 4 näher erläutert wird. Die Verriegelungseinrichtung 60 umfasst einen Riegel 61 mit einer Isolierkappe 62 aus einem elektrisch isolierenden Material an einem aus dem Gehäusekörper 51 herausragenden Ende. Der Riegel 61 weist eine äußere Form auf, die zumindest abschnittsweise an den Querschnitt der Querbohrung 63 im Gehäusekörper 51 angepasst ist. An einem von der Isolierkappe 62 abgewandten Ende des Riegels 61 ist eine Feder 64 oder ein anderes elastisches Element angeordnet, welche den Riegel 61 in Richtung der Isolierkappe 62 drückt. In einem mittleren Abschnitt weist der Riegel 61 zumindest näherungsweise die Form eines Quaders mit einer Öffnung 65 auf. Bei der in Figur 2 dargestellten Position des Riegels 61 liegt dieser an der Konuseinrichtung 52 an, die etwas in die Querbohrung 63 hineinragt. Dadurch wird die Feder 64 an einer weiteren Expansion gehindert.

Eine Welle 70 mit einer Symmetrie- und Rotationsachse 71 ist in der Längsbohrung 72 im Gehäusekörper 51 angeordnet. Die Symmetrie- und Rotationsachse 71 der Welle 70 ist gleichzeitig Symmetrieachse der Konuseinrichtung 52. Wenn die Antriebseinheit 50 an dem oben anhand der Figur 1 dargestellten medizinischen Resektor 10 angeordnet ist, entspricht die Rotationsachse 71 der Welle 70 der Rotationsachse der rotierbaren Hochfrequenz-Elektrode 31, der Welle 32 und der Kupplung 33 oder nimmt zu dieser einen kleinen Winkel ein.

Während die Konuseinrichtung 52 distal des Schiebers 61 der Verriegelungseinrichtung 60 angeordnet ist, ist die Welle 70 proximal des Schiebers 61 angeordnet. In Figur 2 sind die Konuseinrichtung 52 und die Welle 70 jeweils in Richtung parallel zur Rotationsachse 71 von dem Schieber 61 geringfügig beabstandet. Abweichend davon kann die Konuseinrichtung 52 und/oder die Welle 70 unmittelbar am Schieber 61 anliegen.

Am distalen Ende ist die Welle 70 als Kupplungseinrichtung 73 mit einem Hohlraum 74 ausgebildet. Dazu ist die Welle 70 im Bereich der Kupplungseinrichtung 73 aus mehreren Teilen aufgebaut. Der Aufbau und die Form der Welle 70 und insbesondere der Kupplungseinrichtung 73 können jedoch von der Darstellung in Figur 2 abweichen. Der Hohlraum 74 ist zumindest abschnittsweise zylindrisch und weist einen Querschnitt auf, wie er beispielsweise weiter unten mit Bezug auf Figur 6 näher beschrieben wird.

Am distalen Ende der Welle 70 und der Kupplungseinrichtung 73 ist ein O-Ring in einer umlaufenden Nut oder eine andere Dichteinrichtung 75 vorgesehen.

Eine Kontaktierungseinrichtung 80 ist in einem Hohlraum 81 angeordnet. Der Hohlraum 81 umgibt die Kupplungseinrichtung 73 oder grenzt zumindest an diese an. Die Kontaktierungseinrichtung 80 umfasst eine oder mehrere Kontaktfedern 82 mit jeweils einem oder mehreren Schleifkontakten 83. Der oder die Schleifkontakte 83 werden von den Kontaktfedern 82 an eine äußere Mantelfläche 76 der Kupplungseinrichtung 73 gedrückt. Die Kontaktfedern 82 und die Schleifkontakte 83 sind dazu ausgebildet, eine elektrisch leitfähige Verbindung zwischen den ruhenden Kontaktfedern 82 und der rotierenden Kupplungseinrichtung 73 auszubilden. Um eine Oxidation der Oberflächen zu verhindern und eine dauerhaft gute Kontaktierung zu ermöglichen, sind die Mantelfläche 76 der Kupplungseinrichtung und die Schleifkontakte 83 beispielsweise vergoldet.

Figur 3 zeigt eine schematische Darstellung der Antriebseinheit 50, weitgehend entsprechend der Darstellung in Figur 2. Abweichend von Figur 2 ist jedoch bei der in Figur 3 dargestellten Situation eine Kupplungseinrichtung 33, wie sie oben anhand der Figur 1 bereits beschrieben wurde, teilweise in die Konuseinrichtung 52, die Öffnung 65 im Riegel 61 und die Kupplungseinrichtung 73 der Antriebseinheit 50 eingeführt. Dazu ist der Riegel 61 durch Druck auf die Isolierkappe 62 so weit verschoben, dass die Kupplung 33 durch die Öffnung 65 im Riegel 61 hindurch treten kann.

Die Kupplung 33 weist eine umlaufende Nut 34 auf. Am distalen Ende der Kupplung 33 geht diese in die oben anhand der Figur 2 dargestellte Welle 32 über bzw. ist an dieser befestigt. Die Welle 32 umfasst einen elektrisch leitfähigen Kern, beispielsweise einen Stab 35 aus Metall, der mit der Kupplung 33 verschweißt oder verlötet sein kann. Ferner umfasst die Welle einen Isoliermantel 36, der den Stab 35 umschließt und elektrisch isoliert.

Figur 4 zeigt eine weitere schematische Darstellung, die weitgehend den Figuren 3 und 4 entspricht. Im Unterschied zu Figur 3 ist in Figur 4 jedoch eine Situation dargestellt, bei der die Kupplungseinrichtung 33 des medizinischen Resektors so weit in die Kupplungseinrichtung 73 der Antriebseinheit 50 eingeführt ist, dass der Riegel 61 von der Feder 64 in die Nut 34 an der Kupplungseinrichtung 33 des medizinischen Resektors gedrückt wird und in diese eingreift. Der Riegel 61 der Verriegelungseinrichtung 60 verriegelt in der in Figur 4 dargestellten Position die Kupplungseinrichtung 33 des medizinischen Resektors in der Kupplungseinrichtung 73 der Antriebseinheit 50. Diese Verriegelung kann aufgehoben werden, indem der Riegel 61 durch Druck auf die Isolierkappe 62 gegen die Kraft der Feder 64 in die oben anhand der Figur 3 dargestellte Position geschoben wird.

In Figur 4 ist ferner erkennbar, dass die Welle 32 ein Rohr 37 aus Metall oder einem anderen steifen Material aufweist, das den Isoliermantel 36 umschließt. Das Rohr 37 erhöht die Robustheit und die Torsionssteifigkeit und verringert die Elastizität der Welle 32.

Figur 5 zeigt eine schematische Darstellung eines Schnitts durch die gesamte Antriebseinheit 50. Die dargestellte Position der Kupplung 33 des medizinischen Resektors in der Kupplungseinrichtung 73 der Antriebseinheit 50 und die Verriegelung der Kupplungseinrichtung 33 des medizinischen Resektors in dieser Position entsprechen der obigen Darstellung anhand der Figur 4.

Im Unterschied zu den Figuren 2 bis 4 ist in Figur 5 auch eine ebenfalls in den Gehäusekörper 51 der Antriebseinheit 50 integrierte Motoreinrichtung 90 dargestellt. Die Motoreinrichtung 90 umfasst einen Elektromotor 91 und ein Untersetzungsgetriebe bzw. Reduktionsgetriebe 92. Das Reduktionsgetriebe 92 ist über Zahnräder 93, 94, 95 mit der Welle 70 gekoppelt. Dadurch kann der Elektromotor 91 über das Untersetzungsgetriebe 92 und die Zahnräder 93, 94, 95 die Welle 70 und insbesondere die Kupplungseinrichtung 73 der Antriebseinheit 50 sowie die Kupplungseinrichtung 33 und die Welle 32 des mit der Antriebseinheit 50 gekoppelten medizinischen Resektors antreiben.

In Figur 5 ist ferner eine Wand der oben anhand der Figur 1 dargestellten Abflusskammer 14 gezeigt. Es ist erkennbar, dass die Wand 16 an der Konuseinrichtung 52 anliegt. Der Spalt zwischen der Wand 16 und der Konuseinrichtung 52 wird durch die Dichteinrichtung 55 fluiddicht abgedichtet. Durch den Schaft 13 und die Abflusskammer 14 abgesaugtes festes, flüssiges oder gasförmiges Material kann deshalb an dieser Stelle nicht nach außen treten. Die Dichteinrichtung 54 verhindert entsprechend einen Übertritt eines Fluids zwischen der Welle 32 und der Konuseinrichtung 52 hindurch in Richtung zur Verriegelungseinrichtung 60. Der Hohlraum 81 im Gehäusekörper 51, in dem die Kontaktfeder 82 und die Schleifkontakte 83 angeordnet sind, wird durch die Dichteinrichtung 75 nochmals zusätzlich vor dem Eintritt von Fluiden geschützt.

Die gesamte Anordnung ist durch die Integration der Verriegelungseinrichtung 60, der Kupplungseinrichtung 73, der Schleifkontakte 83 auf sehr engem Raum besonders kompakt. Dazu trägt auch bei, dass der Motor 91 und das Getriebe 92 neben statt proximal der Kupplungseinrichtung 73 angeordnet sind.

Figur 6 zeigt schematische Darstellungen von Querschnitten dreier Ausführungsformen der Kupplungseinrichtung 73. Dargestellt ist jeweils ein Schnitt entlang einer Ebene senkrecht zu der oben anhand der Figur 2 dargestellten Rotationsachse 71 der Welle 70 und der Kupplungseinrichtung 73. Die Schnittebene liegt beispiselsweise im Bereich der Schleifkontakte 83.

Bei dem in Figur 6 links dargestellten Beispiel weist der Hohlraum 74 einen halbkreisförmigen Querschnitt auf. Der Rand des halbkreisförmigen Querschnitts weist einen in punktierter Linie dargestellten geraden Abschnitt 77 auf, der in der Mitte von der Rotationsachse 71 der Welle 70 und der Kupplungseinrichtung 73 geschnitten wird. Der gerade Abschnitt 77 des Rands des Querschnitts des Hohlraums 74 ist damit punktsymmetrisch zu seinem Schnittpunkt mit der Rotationsachse 71 der Kupplungseinrichtung 73.

Bei dem in Figur 6 in der Mitte dargestellten Beispiel weist der Hohlraum 74 einen Querschnitt mit einem in punktierter Linie dargestellten S-förmigen Abschnitt 78 auf, der punktsymmetrisch zum Schnittpunkt der Rotationsachse 71 und des Rands des Querschnitts 74 ist, insbesondere punktsymmetrisch zum Schnittpunkt der Rotationsachse 71 und des S-förmigen Abschnitts 78 des Rands des Querschnitts.

Bei dem in Figur 6 rechts dargestelltem Beispiel weist der Hohlraum 74 einen rechteckigen Querschnitt auf, der zur Rotationsachse 71 symmetrisch ist. Neben den in Figur 6 dargestellten Kupplungsformen sind auch andere möglich.

### Bezugszeichen

- 10: medizinischer Resektor
- 11: proximales Ende des medizinischen Resektors 10
- 12: distales Ende des medizinischen Resektors 10
- 13: Schaft des medizinischen Resektors 10
- 14: Abflusskammer am proximalen Ende 11 des medizinischen Resektors 10
- 15: Anschluss für Saugeinrichtung
- 16: Wand der Abflusskammer 14
- 20: Endoskop
- 21: Schaft des Endoskops 20
- 22: Griff am Schaft 21
- 23: Okular des Endoskops
- 24: Kupplung zur Verbindung des Endoskops 20 mit einer Lichtquelle
- 31: rotierbare Hochfrequenz-Elektrode
- 32: Welle
- 33: Kupplungseinrichtung
- 35: Stab
- 36: Isoliermantel
- 37: Rohr
- 40: Krageneinrichtung
- 50: Antriebseinheit
- 51: Gehäusekörper der Antriebseinheit 50
- 52: Konuseinrichtung
- 53: Konusfläche
- 54: erste Dichteinrichtung
- 55: zweite Dichteinrichtung
- 60: Verriegelungseinrichtung
- 61: Riegel
- 62: Isolierkappe
- 63: Querbohrung im Gehäusekörper 51
- 64: Feder
- 65: Öffnung im Riegel 61
- 70: Welle
- 71: Rotationsachse der Welle 70
- 72: Längsbohrung im Gehäusekörper 51
- 73: Kupplungseinrichtung an der Welle 70
- 74: Hohlraum der Kupplungseinrichtung 73
- 75: dritte Dichteinrichtung
- 76: Mantelfläche der Kupplung 33
- 77: gerader Abschnitt des Rands des Querschnitts des Hohlraums 74
- 78: S-förmiger Abschnitt des Rands des Querschnitts des Hohlraums 74
- 80: Kontaktierungseinrichtung
- 81: Hohlraum im Gehäusekörper 51
- 82: Kontaktfeder
- 83: Schleifkontakt
- 90: Motoreinrichtung der Antriebseinheit 50
- 91: Elektromotor der Motoreinrichtung 90
- 92: Untersetzungsgetriebe der Motoreinrichtung 90
- 93: erstes Zahnrad
- 94: zweites Zahnrad
- 95: drittes Zahnrad

## Patentansprüche

1. Antriebseinheit (50) für eine rotierbare Hochfrequenz-Elektrode (31) eines medizinischen Resektors (10) zum Schneiden, Abtragen oder Koagulieren von menschlichem oder tierischem Gewebe, mit:
einer Motoreinrichtung (90) zum Erzeugen einer Rotationsbewegung;
einer Kupplungseinrichtung (73) zur Übertragung der von der Motoreinrichtung **(90)** erzeugten Rotationsbewegung an eine mit der rotierbaren Hochkequenz-Elektrode (31) gekoppelte Welle (32), wenn die Antriebseinheit (50) mit dem medizinischen Resektor (10) gekoppelt ist,
wobei die Antriebseinheit (50) ausgebildet ist, um an der Kupplungseinrichtung (73) eine Drehfrequenz in einem Drehfrequenzbereich bereitzustellen, der im Bereich von 10 Umdrehungen pro Minute bis 200 Umdrehungen pro Minute enthalten ist,
**dadurch gekennzeichnet, dass**
die Antriebseinheit eine Verriegelungseinriehtung (60) zum Verriegeln einer mit der Welle verbundenen, zur lösbaren mechanischen Kopplung mit der Antriebseinheit ausgebildeten Kupplungseinrichtung (33) des medizinischen Resektors (10) in der Antriebseinheit (50) aufweist, wobei die Verriegelungseinrichtung (60) einen federbelasteten Riegel (61) umfasst, der ausgebildet ist, um in eine Nut (34) an der Welle (32) einzugreifen.

2. Antriebseinheit (50) nach dem vorangehenden Anspruch, bei welcher der Riegel (61) eine Öffnung (65) oder eine Ausnehmung aufweist, in welcher die Welle (32) angeordnet ist, wenn der medizinische Resektor (10) mit der Antriebseinheit (50) gekoppelt ist.

3. Antriebseinheit (50) nach einem der vorangehenden Ansprüche, wobei die Motoreinrichtung (90) ein Reduktionsgetriebe (92) umfasst.

4. Antriebseinheit (50) nach einem der vorangehenden Ansprüche, bei der die Kupplungscinrichtung (73) einen Hohlraum (74) mit einem zylindrischen Abschnitt, der nicht zur Rotationsachse (71) der Kupplungseinrichtung (73) symmetrisch ist, umfasst.

5. Antriebseinheit (50) nach einem der vorangehenden Ansprüche, bei der die Antriebseinheit (50) ausgebildet ist, um an der Kupplungseinrichtung (73) eine Drehfrequenz in einem Drehfrequenzbereich bereitzustellen, der im Bereich von 30 Umdrehungen pro Minute bis 60 Umdrehungen pro Minute enthalten ist.

6. Antriebseinheit (50) nach einem der vorangehenden Ansprüche, bei der die Antriebseinheit (50) ausgebildet ist, um so mit dem medizinischen Resektor (10) mechanisch verbunden zu werden, dass eine Rotationsachse (71) der Kupplungseinrichtung (73) zu einer Längsachse des medizinischen Resektors (10) nicht parallel ist.

7. Antriebseinheit (50) nach einem der vorangehenden Ansprüche,
wobei die Verriegelungseinrichtung (60) zum Verriegeln der Kupplungseinrichtung (33) des medizinischen Resektors (10) in der Antriebseinheit (50) ausgebildet ist, um nicht mit der Kupplungseinrichtung (73) zu rotieren.

8. Antriebseinheit (50) nach einem der vorangehenden Ansprüche, wobei die Kupplungscinrichtung (73) ferner ausgebildet ist, um ein elektrisches HochfrequenzSignal von der Antriebseinheit (50) zu der Welle (32) zu übertragen.

9. Antriebseinheit (50) nach einem der vorangehenden Ansprüche, ferner mit:
einer Kontaktierungseinrichtung (80) zur Übertragung eines elektrischen Hochfrequenz-Signals zu der Kupplungseinrichtung (73), wobei die Kontaktierungseinrichtung (80) einen Schleifkontakt (83) umfasst, der an einer Mantelfläche (76) der Kupplungseinrichtung (73) anliegt.

10. Antriebseinheit (50) nach einem der vorangehenden Ansprüche, wobei die Antriebseinheit (50) ausgebildet ist, mit einer Abflusskammer (14) unmittelbar, fluiddicht und lösbar verbunden zu werden.

11. Medizinischer Resektor (10) zum Schneiden, Abtragen oder Koagulieren von menschlichem oder tierischem Gewebe, mit:
einer rotierbaren Hochfrequenz-Elelctrode (31) am distalen Ende (12) des medizinischen Resektors (10);
einer Kupplungseinrichtung (33) am proximalen Ende (11) des medizinischen Resektors (10);
einer Welle (32), welche die Kupplungseinrichtung (33) mit der Hochfrequenz-Elektrode (31) verbindet,
wobei die Kupplungseinrichtung (33) zur lösbaren mechanischen Kapplung mit einer Antriebseinheit (50) ausgebildet ist, die eine Drehfrequenz in einem Drehfrequenzbereich bereitstellt, der im Bereich von 10 Umdrehungen pro Minute bis 200 Umdrehungen pro Minute enthalten ist,
**dadurch gekennzeichnet, dass**
die Welle eine Not aufweist, in die ein federbelasteter Riegel einer Verriegelungseinrichtung der Antriebseinheit eingreifen kann, um die Kupplungseinrichtung des medizinischen Resektors in der Antriebseinheit zu verriegeln.

12. Medizinischer Resektors (10) nach Anspruch 12, wobei die Kupplungseinrichtung (33) einen zylindrischen Abschnitt umfasst, der nicht zur Rotationsachse der Kupplungseinrichtung (33) symmetrisch ist.

13. Medizinischer Resektor (10) nach Anspruch 12 oder 13, bei dem die Kupplungseinrichtung zur lösbaren mechanischen Koppelung mit einer Antriebseinheit (50) ausgebildet ist, die eine Drehfrequenz in einem Drehfrequenzbereich bereitstellt, der im Bereich von 30 Umdrehungen pro Minute bis 60 Umdrehungen pro Minute enthalten ist.

14. Medizinischer Resektor (10) nach einem der Ansprüche 12 bis 14, bei dem die Welle (32) eine Rotationsachse aufweist, die zu einer Längsachse des medizinischen Resektors (10) nicht parallel ist.

## Claims

1. Drive unit (50) for a rotatable radiofrequency electrode (31) of a medical resector (10) for cutting, ablating or coagulating human or animal tissue, comprising:
a motor device (90) for generating a rotational movement;
a coupling device (73) for transmitting the rotational movement generated by the motor device (90) to a shaft (32) coupled to the rotatable radiofrequency electrode (31) if the drive unit (50) is coupled to the medical resector (10),
the drive unit (50) being configured to provide a rotational frequency in a rotational frequency range at the coupling device (73), which rotational frequency range is contained in the range from 10 revolutions per minute to 200 revolutions per minute,
**characterized in that**
the drive unit comprises a locking device (60) for locking a coupling device (33), connected to the shaft and configured for detachable mechanical coupling to the drive unit, of the medical resector (10) in the drive unit (50), the locking device (60) comprising a spring-loaded bolt (61), which is configured to engage in a groove (34) on the shaft (32).

2. Drive unit (50) according to the preceding claim, in which the bolt (61) has an opening (65) or a recess, in which the shaft (32) is arranged if the medical resector (10) is coupled to the drive unit (50).

3. Drive unit (50) according to one of the preceding claims, wherein the motor device (90) comprises a step-down gear (92).

4. Drive unit (50) according to one of the preceding claims, in which the coupling device (73) comprises a cavity (74) with a cylindrical section which is not symmetrical with respect to the rotational axis (71) of the coupling device (73).

5. Drive unit (50) according to one of the preceding claims, in which the drive unit (50) is configured to provide a rotational frequency in a rotational frequency range at the coupling device (73), which rotational frequency range is contained in the range from 30 revolutions per minute to 60 revolutions per minute.

6. Drive unit (50) according to one of the preceding claims, in which the drive unit (50) is configured to be connected mechanically to the medical resector (10) in such a way that a rotational axis (71) of the coupling device (73) is not parallel to a longitudinal axis of the medical resector (10).

7. Drive unit (50) according to one of the preceding claims, wherein the locking device (60) is configured to lock the coupling device (33) of the medical resector (10) in the drive unit (50) so as not to rotate with the coupling device (73).

8. Drive unit (50) according to one of the preceding claims, wherein the coupling device (73) is furthermore configured to transmit an electrical radiofrequency signal from the drive unit (50) to the shaft (32).

9. Drive unit (50) according to one of the preceding claims, furthermore comprising:
a contacting device (80) for transmitting an electrical radiofrequency signal to the coupling device (73), the contacting device (80) comprising a sliding contact (83), which rests against a barrel surface (76) of the coupling device (73).

10. Drive unit (50) according to one of the preceding claims, wherein the drive unit (50) is configured to be connected directly to an outflow chamber (14) in a fluid-tight and detachable fashion.

11. Medical resector (10) for cutting, ablating or coagulating human or animal tissue, comprising:
a rotatable radiofrequency electrode (31) at the distal end (12) of the medical resector (10);
a coupling device (33) at the proximal end (11) of the medical resector (10);
a shaft (32), which connects the coupling device (33) to the radiofrequency electrode (31),
the coupling device (33) being configured for detachable mechanical coupling to a drive unit (50) which provides a rotational frequency in a rotational frequency range which is contained in the range from 10 revolutions per minute to 200 revolutions per minute,
**characterized in that**
the shaft has a groove, into which a spring-loaded bolt of a locking device of the drive unit can engage in order to lock the coupling device of the medical resector in the drive unit.

12. Medical resector (10) according to Claim 12, wherein the coupling device (33) comprises a cylindrical section which is not symmetrical to the rotational axis of the coupling device (33).

13. Medical resector (10) according to Claim 12 or 13, in which the coupling device is configured for detachable mechanical coupling to a drive unit (50) which provides a rotational frequency in a rotational frequency range which is contained in the range from 30 revolutions per minute to 60 revolutions per minute.

14. Medical resector (10) according to one of Claims 12 to 14, in which the shaft (32) has a rotational axis which is not parallel to a longitudinal axis of the medical resector (10).

## Revendications

1. Unité d'entraînement (50) pour électrode rotative (31) à haute fréquence d'un résecteur médical (10) destiné à couper, enlever ou coaguler des tissus humains ou animaux, et présentant
un dispositif de moteur (90) qui forme un déplacement de rotation,
un dispositif d'accouplement (73) qui transfère le déplacement de rotation formé par le dispositif de moteur (90) à un arbre (32) accouplé à l'électrode rotative (31) à haute fréquence lorsque l'unité d'entraînement (50) est accouplée au résecteur médical (10),
l'unité d'entraînement (50) est configurée pour délivrer au dispositif d'accouplement (73) une fréquence de rotation située dans une plage de fréquence de rotation incluse dans la plage de 10 tours par minute à 200 tours par minute,
**caractérisée en ce que**
l'unité d'entraînement présente un dispositif de verrouillage (60) qui verrouille un dispositif d'accouplement (33) du résecteur médical (10), relié à l'arbre et configuré pour assurer un accouplement mécanique libérable avec l'unité d'entraînement dans l'unité d'entraînement (50), le dispositif de verrouillage (60) comportant un verrou (61) sollicité par ressort configuré pour s'engager dans une rainure (34) de l'arbre (32).

2. Unité d'entraînement (50) selon la revendication précédente, dans laquelle le verrou (61) présente une ouverture (65) ou une découpe dans laquelle l'arbre (32) est disposé lorsque le résecteur médical (10) est accouplé à l'unité d'entraînement (50).

3. Unité d'entraînement (50) selon l'une des revendications précédentes, dans laquelle le dispositif de moteur (90) comporte une transmission réductrice (92).

4. Unité d'entraînement (50) selon l'une des revendications précédentes, dans laquelle le dispositif d'accouplement (73) comporte une cavité (74) dotée d'une partie cylindrique non symétrique par rapport à l'axe de rotation (71) du dispositif d'accouplement (73).

5. Unité d'entraînement (50) selon l'une des revendications précédentes, dans laquelle l'unité d'entraînement (50) est configurée pour délivrer au dispositif d'accouplement (73) une fréquence de rotation située dans une plage de fréquences de rotation incluse dans la plage de 30 tours par minute à 60 tours par minute.

6. Unité d'entraînement (50) selon l'une des revendications précédentes, dans laquelle l'unité d'entraînement (50) est configurée pour être reliée mécaniquement au résecteur médical (10) de telle sorte que l'axe de rotation (71) du dispositif d'accouplement (73) ne soit pas parallèle à l'axe longitudinal du résecteur mécanique (10).

7. Unité d'entraînement (50) selon l'une des revendications précédentes, dans laquelle le dispositif de verrouillage (60) qui sert à verrouiller le dispositif d'accouplement (33) du résecteur médical (10) dans l'unité d'entraînement (50) est configuré de manière à ne pas tourner avec le dispositif d'accouplement (73).

8. Unité d'entraînement (50) selon l'une des revendications précédentes, dans laquelle le dispositif d'accouplement (73) est en outre configuré pour transférer à l'arbre (32) un signal électrique à haute fréquence provenant de l'unité d'entraînement (50).

9. Unité d'entraînement (50) selon l'une des revendications précédentes, dotée en outre d'un dispositif (80) de mise en contact qui transfère un signal électrique à haute fréquence au dispositif d'accouplement (73), le dispositif (80) de mise en contact comportant un contact coulissant (83) qui repose contre la surface d'enveloppe (76) du dispositif d'accouplement (73).

10. Unité d'entraînement (50) selon l'une des revendications précédentes, dans laquelle l'unité d'entraînement (50) est configurée pour être reliée de manière directe, étanche aux fluides libérables à une chambre d'évacuation (14).

11. Résecteur médical (10) destiné à découper, enlever ou coaguler les tissus humains ou animaux et présentant :
une électrode rotative (31) à haute fréquence disposée à l'extrémité distale (12) du résecteur médical (10),
un dispositif d'accouplement (33) situé à l'extrémité proximale (11) du résecteur médical (10),
un arbre (32) qui relie le dispositif d'accouplement (33) à l'électrode (31) à haute fréquence,
le dispositif d'accouplement (33) étant configuré pour être accouplé mécaniquement de manière libérable à une unité d'entraînement (50) qui délivre une fréquence de rotation dans la plage de fréquences de rotation incluse dans la plage de 10 tours par minute à 200 tours par minute,
**caractérisé en ce que**
l'arbre présente une rainure dans laquelle un verrou sollicité par ressort d'un dispositif de verrouillage de l'unité d'entraînement peut s'engager pour verrouiller le dispositif d'accouplement du résecteur médical dans l'unité d'entraînement.

12. Résecteur médical (10) selon la revendication 12, dans lequel le dispositif d'accouplement (33) comporte une partie cylindrique qui n'est pas symétrique à l'axe de rotation du dispositif d'accouplement (33).

13. Résecteur médical (10) selon la revendication 12 ou 13, dans lequel le dispositif d'accouplement est configuré pour être accouplé mécaniquement de manière libérable à un unité d'entraînement (50) qui délivre une fréquence de rotation située dans une plage de fréquences de rotation incluse dans la plage de 30 tours par minute à 60 tours par minute.

14. Résecteur médical (10) 12 à 14, dans lequel l'arbre (32) présente un axe de rotation qui n'est pas parallèle à l'axe longitudinal du résecteur médical (10).
